(19) Europäisches Patentamt
European Patent Office
Office européen des brevets

(11) **EP 0 527 778 B1**

(12) **EUROPEAN PATENT SPECIFICATION**

(45) Date of publication and mention
of the grant of the patent:
**19.06.1996 Bulletin 1996/25**

(21) Application number: **91907916.0**

(22) Date of filing: **11.03.1991**

(51) Int Cl.6: **C12N 15/62**, C07K 1/00,
C07K 14/00, C12P 21/02

(86) International application number:
**PCT/US91/01543**

(87) International publication number:
**WO 91/15589 (17.10.1991 Gazette 1991/24)**

(54) **IMPROVED PROCESS OF PURIFYING RECOMBINANT PROTEINS AND COMPOUNDS USEFUL IN SUCH PROCESS**

VERFAHREN ZUR REINIGUNG VON REKOMBINANTEN PROTEINEN UND DAFÜR NÜTZLICHE PRODUKTE

PROCEDE AMELIORE DE PURIFICATION DE PROTEINES DE RECOMBINAISON ET PROCEDES UTILES DANS LEDIT PROCEDE

(84) Designated Contracting States:
**AT BE CH DE DK ES FR GB GR IT LI LU NL SE**

(30) Priority: **09.04.1990 US 506605**

(43) Date of publication of application:
**24.02.1993 Bulletin 1993/08**

(73) Proprietor: **THE UPJOHN COMPANY**
**Kalamazoo, Michigan 49001 (US)**

(72) Inventor: **SHARMA, Satish, K.**
**Portage, MI 49002 (US)**

(74) Representative: **Perry, Robert Edward et al**
**GILL JENNINGS & EVERY**
**Broadgate House**
**7 Eldon Street**
**London EC2M 7LH (GB)**

(56) References cited:
**EP-A- 184 355**          **EP-A- 282 042**
**WO-A-88/10299**

- **JOURNAL OF BIOLOGICAL CHEMISTRY, vol. 263, no. 15, 25 May 1988, Baltimore, MD (US); M.C. SMITH et al., pp. 7211-7215 :"Chelating peptide-immobilized metal ion affinity chromatography"**

**Description**

FIELD OF THE INVENTION

The present invention relates to a method of obtaining a purified desired protein product by producing it as a recombinant fusion protein in a host, purifying the fusion protein and cleaving it into constitutive proteins.

BACKGROUND OF THE INVENTION

The rapid developments in recombinant DNA methodology have allowed the production of polypeptides, proteins, and their analogs in unlimited quantities in a very short period of time. These developments have created a need to handle purification of these proteins from complex mixtures in highly efficient and predictable manners.

Recombinant DNA technology may be used for the production of desired polypeptides and proteins in host cells. Genes for desired proteins may be isolated from the genetic material of cells which contain the gene in nature or they may be chemically synthesized. The isolated or chemically synthesized gene may be inserted and expressed in host cell systems which produce protein products at high levels.

The desired protein products must then be isolated and recovered from the total amount of protein produced by the host cells. The purification of heterologous polypeptides produced by host cells can be very expensive and can cause denaturation of the protein product itself. An overview of protein purification techniques is provided in the Background Art section of U.S. Patent Number 4,782,137 issued Nov. 1, 1988 to Hopp et al, which is incorporated herein by reference. Among the methods to purify proteins that are described in Hopp, the most commonly practiced are ion-exchange, hydrophobic chromatography, and gel filtration. The major disadvantage of these approaches are the lack of specificity of each technique. Thus, these techniques are unsuitable to achieve pure protein in high yields. Even small changes in amino acid composition may change the purification properties. A modified purification procedure needs to be developed and optimized for each new protein. In the case of rDNA derived proteins, structural and functional consequences of heterologous gene expression (H. Bialy, Bio/technology, 5:884, 1987) are additional factors that may make it impossible to predict selection of these purification methods for a given protein.

Desired protein molecules may be isolated from complex mixtures by methods based on solubility differences. For example, isoelectric precipitation makes use of the alteration in protein solubility as a function of pH while fractionation with a solvent is based on the variation in protein solubility as a function of dielectric constant. Neutral salts, for example, ammonium sulfate, are employed to precipitate proteins due to decreased protein solubility based on high ionic strength of the salt. The drawback is that solvent fractionation can cause protein denaturation. Neither of these methods are capable of purifying proteins beyond a moderate level.

To avoid the negative elements of above techniques, affinity chromatography is often preferred. It is based on the ability of proteins to bind non-covalently but specifically with an immobilized ligand. When used alone, it can purify proteins from complex mixtures without significant loss. It requires the availability of the corresponding ligand for the desired protein; for example, an antibody for a protein antigen. It should be stressed that in some cases it may be difficult to obtain a specific ligand and such ligands do not exist for all proteins. As a result, this technique has not been applied as a universal method for protein purification.

To circumvent this limitation, recombinant DNA technology may be used to provide an affinity purification system where antibodies to a linker peptide may be used as an immunoaffinity ligand. This should provide a method that is capable of purifying recombinant proteins in one-step, using affinity chromatography, without sacrificing high yields. The Hopp patent relates to synthesis of a fusion peptide containing an antigenic linker peptide. The fusion peptide of Hopp is passed through a column containing immobilized antibodies which bind to the antigenic linker. Thus, the fusion protein may be isolated. The major drawbacks of this technique are that either the buffer conditions which are necessary to allow immunogenic complexing or the buffer conditions which must be present to terminate such complexes may denature the desired polypeptide product.

Immobilized Metal Ion Affinity Chromatography (IMAC) for fractionating proteins was first disclosed by Porath, J. et al., Nature 258:598-599 (1975). Porath disclosed derivatizing a resin with iminodiacetic acid (IDA) and chelating metal ions to the IDA-derivatized resin. Porath disclosed that proteins could be immobilized in a column which contained immobilized metal ions. The teachings of Porath include attaching a commonly used iminodiacetic acid (IDA) to a matrix followed by chelating a metal ion to the IDA-containing support resin. The proteins bind to the metal ion(s) through amino acid residues capable of donating electrons. Amino acids with potential electron donor groups are cysteine, histidines, and tryptophan. Proteins interact with metal ions through one or more of these amino acids with electron-donating side chains. The actual mechanisms which result in binding of proteins to free metal ions or immobilized metal ions are not well known. A number of factors play a role; for example, conformation of the particular protein, number of available coordination sites on the immobilized metal ion, accessibility of protein side chains to the metal ion, number of available amino acids for coordination with the immobilized metal ion. Therefore, it is difficult to predict which protein will bind an with what affinity.

US-A-4,569,794 discloses that certain amino acid residues are responsible for the binding of the protein to the immobilized metal ions. However, if histidine side chains are involved in the binding the bound protein can be eluted by lowering the pH or using competitive counter ligands such as imidazole. Histidines containing di- or tripeptides in proteins were used to show that IMAC is a specific and selective purification technique. Accordingly, US-A-4569794 discloses using recombinant DNA techniques to attach a metal chelating peptide to a desired polypeptide reproduced by recombinant techniques in order to provide a handle to the desired polypeptide. This handle can be used in protein purification by providing the chimeric protein with a metal chelating linker. US-A-4569794 discloses several examples of metal-chelating peptides, specifically those containing histidine, cysteine, methionine, glutamic acid, aspartic acid, lysine, and tyrosine. US-A- 4569794 also discloses that a fusion protein comprising a desired polypeptide with an attached metal chelating peptide handle may be purified from contaminants by passing the fusion protein and contaminants through columns containing immobilized metal ions. The fusion protein will chelate at the metal chelating peptide linker to the immobilized metal ions. The contaminants freely pass through the column and can thus be removed. By changing the conditions of the column, the chimerics can be released and then can be collected in pure form.

EP-A-0282042 discloses fusion proteins and a process for their purification using metal-chelating affinity chromatography based on NTA-resins. Various fusion proteins are disclosed, including those having 2 to 6 consecutive His residues in the affinity peptide of a fusion protein.

## SUMMARY OF THE INVENTION

A novel fusion protein according to this invention is represented by the formula

$$R_1\text{-}(His\text{-}X)_n\text{-}R_2$$

wherein $(His\text{-}X)_n$ represents a metal-chelating peptide;

$R_1\text{-}(His\text{-}X)_n$ includes a dipeptidyl peptidase I substrate;

$n = 3$ to $6$;

each X is independently selected from Asp, Glu, Ala, Gly, Val, Ser, Leu, Ile and Thr;

$R_1$ is hydrogen or an even number of amino-acids free of Pro residues and not containing Arg or Lys residues at the odd-numbered positions; and

$R_2$ is a desired polypeptide or a desired polypeptide indirectly covalently linked to the metal-chelating peptide.

According to a further aspect of the invention, a fusion protein as defined above may be purified by the steps of:

(a) contacting immobilised metal ions with cell extract material which contains cellular proteinaceous molecules including the fusion protein, wherein the fusion protein forms an affinity bond to the metal ions;

(b) removing cell extract material not bonded to the metal ions;

(c) eliminating the affinity bond; and

(d) collecting purified fusion protein.

According to another aspect of the present invention, a kit for use in purifying a fusion protein as defined above comprises:

(a) a DNA molecule which encodes the fusion protein; and

(b) a column containing immobilised metal ions.

The process of the present invention allows recombinant polypeptides and proteins to be purified efficiently, and recovered in pure biologically-active form. The metal-binding peptide may be removed from the purified chimeric protein without having to introduce a site-specific cleavage sequence.

When the fusion protein is produced, the desired protein may be isolated and purified by passing the fusion protein through a column containing immobilised metal ions. The fusion protein chelates to the immobilised metal ions for a sufficient amount of time to allow it to be separated from other materials. It is possible to employ the commonly used IDA resin in IMAC for the purification of the novel recombinant proteins having at least three alternating histidine residues. The metal-chelating peptide according to the present invention may be removed from the desired protein portion by dipeptidylpeptidase I digestion.

The conditions needed in the purification step of the present method do not denature the fusion protein. The fusion protein may thus be purified to a biologically active final product in high yield using relatively few steps.

## DETAILED DESCRIPTION OF THE INVENTION

The present invention relates to the purification of biologically active recombinant polypeptides and/or proteins produced by transformed host cells. The desired biologically active recombinant proteins are most preferably produced in a soluble form or secreted from the host. According to the present invention, the desired biologically active protein is expressed as a fusion protein containing a metal ion chelating peptide that can be immobilized by an immobilized resin that contains metal ions. The fusion protein is purified from the material present in the secretion media or extraction solution it is contained in by contacting the secretion media or extraction solution with the metal ion resin. A proteolytic cleavage site may be optionally interposed between the portion of the fusion protein which contains the desired protein and the peptide linker whereby an enzyme capable of cleaving the fusion protein at the recognition site can be used to separate the components. Moreover, optionally, the peptide linker portion of the fusion protein is a substrate for dipeptidlypeptidase (DPP) digestion, specifically DPP I. The DPP I can be added to the purified fusion protein to remove the peptide linker portion and thereby produce the desired protein.

The desired proteins which are produced by recombinant DNA technology and purified are expressed as a fusion protein. The fusion protein is produced by host cells transformed with the genetic information encoding the fusion protein. The host cells may secrete the fusion protein into the culture media or store it in the cells whereby the cells must be collected and disrupted in order to extract the product. As hosts, both E. coli and mammalian cells are preferred hosts. Of these two, E. coli is the preferred host according to this invention.

The culture media containing the secreted fusion protein or the cell extracts containing the fusion protein are passed through a column that contains an immobilized resin comprising a metal ion. The preferred metal ions are copper, zinc, cobalt, and nickel; nickel being the most preferred. All of the components of either solution freely pass through the column except the fusion protein. The immobilized resin chelates the peptide linker and, therefore, impedes the movement of the fusion protein through the column. Thus, all the impurities are eluted through the column except the fusion protein. The conditions in the column can then be changed so that the resin releases the fusion protein which can then be collected. The bound chimeric protein molecules could be eluted by pH change, imidazole, or competition with another linker peptide from the IMAC column, and the chelating peptide cleaved from the chimeric protein by site specific proteolysis, thus releasing the highly purified protein molecule.

It is to be appreciated that some polypeptide or protein molecules will possess the desired enzymatic or biological activity with the metal chelate peptide still attached either at the C-terminal end or at the N-terminal end or both. In those cases the purification of the chimeric will be accomplished after the IMAC column, without subjecting the protein into site-specific proteolysis or DPP I cleavage.

The purification process of the present invention can be used batchwise or in continuously run columns.

After the cleavage reaction occurs, the desired protein is generated from the fusion protein. These desired proteins may be recovered using simple techniques well known to those having ordinary skill in the art.

The terms "fusion protein" and "chimeric proteins" as used herein are interchangeable and refer polypeptides and and proteins which consist of one or two metal ion chelating linker peptides and a biologically active polypeptide or protein or a short peptide linked directly or indirectly to the linker peptides.

The term "desired polypeptide" used herein refers to a biologically active polypeptide or protein.

The terms "metal ion chelating peptide", "metal binding peptide" and "linker peptide" are used interchangeably to refer to an amino acid sequence which displays an affinity to metal ions. The minimum length of the immobilized metal ion chelating peptide according to the present invention is six amino acids including three alternating histidines. The most preferred length is twelve amino acids including six alternating histidines.

The term "enzyme" referred to herein in the context of a cleavage enzyme means a polypeptide or protein which recognizes a specific amino acid sequence in a polypeptide and cleaves the polypeptide at the scissile bond. In the present invention, DPP I is the enzyme. DPP I cleaves its substrate by removing two amino acids at a time from the N-terminus.

The terms "cleavage site" used herein refers to an amino acid sequence which is recognized and cleaved by an enzyme or chemical means at the scissile bond.

The term "scissile bond" referred to herein is the juncture where cleavage occurs.

The term "dipeptidylpeptidase I substrate" refers to an amino acid sequence which is free of Proline residues and does not contain Arginine or Lysine residues at odd numbered positions.

The term "odd numbered positions" refers to the amino acids residues occupying odd numbered sites on the amino acid sequence of a peptide. The odd numbered positions are determined from the N-terminus of the peptide and comprise amino acids 1, 3, 5, . . . etc.

The present invention may be used to purify any prokaryotic or eukaroytic protein that can be expressed as the product of recombinant DNA technology in a transformed host cell. These recombinant protein products include hormones, receptors, enzymes, storage proteins, blood proteins, mutant proteins produced by protein engineering techniques, or synthetic proteins.

The chimeric proteins of this invention are prepared by recombinant DNA methodology. In accordance with the present invention, a gene sequence coding for a desired protein is isolated, synthesized or otherwise obtained and operably linked to a DNA sequence coding for the linker peptide. The hybrid gene containing the gene for a desired protein operably linked to a DNA sequence encoding a linker peptide is referred to as a chimeric gene.

The chimeric gene is inserted into an expression vector which allows for the expression of the desired chimeric protein in a suitable transformed host. The expression vector provides the inserted chimeric gene with the necessary regulatory sequences to control expression in the suitable transformed host.

There are six elements of control expression sequence for proteins which are to be secreted from a host into the medium, while five of these elements apply to chimeric proteins expressed intracellularly. These elements in the order they appear in the gene are: a) the promoter region; b) the 5' untranslated region; c) signal sequence; d) the chimeric coding sequence; e) the 3' untranslated region; f) the transcription termination site.

Fusion protein which are not secreted do not contain c), the signal sequence.

Methods and materials for preparing recombinant vectors and transforming host cells using the same, replicating the vectors in host cells and expressing biologically active foreign polypeptides and proteins are described in Principles of Gene Manipulation, by Old and Primrose, 2nd edition, 1981 and Maniatis et al., Molecular Cloning, Cold Spring Harbor Laboratory 1982, both incorporated herein by reference.

In addition to the genetic information necessary to encode and produce the fusion peptide, a column containing an immobilized resin is provided. Immobilized resins used in the present invention are well known in the art.

Additionally, the present invention relates to genes which encode fusion proteins, expression vectors containing the same, microorganisms transformed with these expression vectors, and process for obtaining these genes, expression vectors, and microorganisms transformed with said vectors.

According to the present invention, the metal ions may be copper, nickel, cobalt, or zinc. According to the present invention, the desired polypeptides produced may include HIV-1 RNase H, tPA, IL-1, BST, IL-1 receptor, CD4, HIV RT, human nerve growth factor, sCD4-PE40, human respiratory syncytial virus (RSV) FG chimeric glycoprotein, and PE40.

The metal ion chelating linker peptides in accordance with this invention are defined by the general formula:

$$R_1\text{-}(His\text{-}X)_n\text{-}R_2$$

wherein $(His\text{-}X)_n$ represents a metal-chelating peptide;

$R_1\text{-}(His\text{-}X)_n$ includes a dipeptidyl peptidase I substrate;
n = 3 to 6;
each X is independently selected from Asp, Glu, Ala, Gly, Val, Ser, Leu, Ile and Thr;
$R_1$ is hydrogen or an even number of amino-acids free of Pro residues and not containing Arg or Lys residues at the odd-numbered positions; and
$R_2$ is a desired polypeptide or a desired polypeptide indirectly covalently linked to the metal-chelating peptide.

A preferred sub-class of x is Asp and Glu; Asp is most preferred.

In the preferred embodiment of the present invention, a column is provided that contains immobilized resin made up of nickel ions which recognizes the above described linker peptides.

$R_1$ may be a second desired polypeptide or a polypeptide which drives expression. If $R_1$ is a second desired polypeptide it may or may not have a cleavage site interposed between it and the linker peptide.

As noted above, $R_1$ may be a protein which drives expression. It is well known that the presence of some proteins in a cell result in expression of genes. If a chimeric protein contains an active portion of the protein which prompts or enhances expression of the gene encoding it, greater quantities of the protein may be expressed than if it were not present.

The peptide linker portion is a dipeptidylpeptidase I substrate, that is it does not contain any Proline residues. Accordingly, in a fusion protein according to this embodiment of the present invention, R1 does not comprise Pro and X is not Pro. In the preferred form of this embodiment, the 2nd or 3rd amino acid of the desired protein is Proline since DPP I is unable to digest if Proline is involved in a cleavage site. Thus, when the entire peptide linker portion is removed, digestion by DPP I will stop and the complete desired protein will be left.

Examples of fusion proteins having a metal chelating peptide between two polypeptides can be represented by the formula:

$$Y \text{ - } mbp \text{ - } Z$$

wherein Y and Z are desired polypeptides and mbp is a metal chelating peptide. In such cases, Y and Z can both be desired polypeptides. Alternatively, one of the two can be a polypeptide which drives gene expression. In either case Y, Z or both can have an adjacent cleavage site for post purification processing.

Specific constructions according to the formula Y-mbp-Z can be made by those having ordinary skill in the art using standard techniques.

Example  Fusion Proteins Comprising the Desired Protein and a Peptide Linker that is a Substrate for DPP I

A strategy to purify DPP I-cleavable chimeric proteins from recombinant E. coli is described. It is based on a genetically engineered N-terminal extension with specificity both for a metal ion for purification and for DDP-I for cleavage of the same extension to generate the desired protein which contains Pro at position 2 and/or at position 3 as a stop signal. These are designed such that there are no prolines in the entire linker portion, no lysines or arginines at odd numbered positions in the linker and at least three alternating histidine residues. The stop signal for cleavage is provided by the 2nd and/or 3rd proline of the desired protein. Fusion proteins can comprise the above-described N-terminal extension linked to the HIV-1 trans-activator (Tat) protein as the desired protein portion. In such a fusion protein, DPP I cleavage can remove the metal chelating peptide (mcp), thereby liberating the desired protein.

The preferred DPP I cleavable N-terminal extensions according to the present invention are outlined as follows:

Fusion protein containing HIV-1 trans-activator (Tat) and an N-terminal extension (mcp#1-Seq ID 1) linked to Tat (Tat/mcp#1):

Met-Glu-His-Glu-His-Glu-His-Glu-His-Glu-Tat
Fusion protein containing HIV-1 trans-activator (Tat) and an N-terminal extension (mcp#2-Seq ID 2) linked to Tat (Tat/mcp#2):
Met-Glu-His-Asp-His-Asp-His-Asp-His-Asp-Tat
Fusion protein containing HIV-1 trans-activator (Tat) and an N-terminal extension (mcp#3-SEQ ID 3) linked to HIV-1 Tat (Tat/mcp#3):
Met-Ile-His-Asp-His-Asp-His-Asp-His-Asp-Tat

The N-terminal methionine is a consequence of protein biosynthesis in E. coli. It is not processed when Ile or Glu is the next amino acid.

Chimeric genes which encode these fusion proteins are constructed, inserted into and expressed in E. coli. Expressed proteins are purified using S-Sepharose chromatography and C-4 Reverse Phase HPLC. N-terminal sequencing is used to characterize the fusion proteins. Application of the alternating histidine-containing fusion proteins to the purification of recombinant proteins by IMAC and subsequent removal of the N-terminal extension by DPP I confirm the utility of the present invention.

Construction of Chimeric Genes Containing HIV-1 Tat Gene

All recombinant DNAs are prepared by standard techniques. Oligonucleotides corresponding to the metal chelating peptide/DPP I substrate sequence are constructed, purified, annealed and ligated to a gene encoding HIV-1 Tat (Tat) to form a chimeric gene. The Tat gene expression is described in Hasler, J.M., et al. (AIDS Research and Human Retroviruses 5 (1989) pp. 507-515). The chimeric gene is constructed using techniques well known to those having ordinary skill in the art.

To prepare expression vectors encoding a fusion protein comprising a linker that is a DPP I substrate and has alternating Histidines linked with the gene encoding Tat, a chimeric gene is constructed and inserted into an expression vector. Expression vectors containing the chimeric gene constructs are used to transform E. coli and expression of the genes in E. coli results in the production of the fusion proteins encoded by the chimeric genes. The molecular biology techniques used to produce and express the chimeric gene are well known to those having ordinary skill in the art. These fusion proteins contain the Tat amino acid sequence and an N-terminal extension that is a DPP I substrate which contains alternate histidines and is therefore a metal chelating peptide. Preparation of Crude E. coli Extracts and Isoiation of Fusion Proteins for Sequencing

Approximately 3 g of E. coli cell paste from cells transformed with the desired expression vector is suspended in 30 ml of 0.25 M potassium phosphate, pH 7.2 containing 1 mM dithiothreitol (DTT), EDTA, phenylmethylsulfonyl fluoride (PMSF), and benzamidine HCL, 10 mg/liter aprotinin, leupeptin, and bestatin. This suspension is passed through a French Press three times to break the cells. Cell lysates are centrifuged at 12,000 rpm for 1 hr. The supernatant is removed and solid ammonium sulfate added to 40% saturation. After stirring for 1 hr, the suspension is centrifuged at 12,000 rpm for 1 hr. The supernatant is discarded and the pellet is redissolved in 2.25 mls of 50 mM Tris pH 7.5 containing 1 mM DTT, PMSF, and benzamidine. The solution is then dialyzed overnight into appropriate buffer for S-Sepharose ion-exchange chromatography followed by reverse phase HPLC. Purified Tat protein is used for characterization by N-terminal sequence analysis.

Purification of Fusion Proteins Using IMAC

The feasibility of using a metal chelating peptide for the purification of recombinant proteins from crude extracts can be demonstrated by using the following chimerics expressed in recombinant E. coli with HIV-1 Tat (Tat) as the model desired protein. Fusion proteins Tat/mcp #1, Tat/mcp #2 and Tat/mcp #3 are each purified.

IMAC columns are prepared as follows. Chelating Sepharose Fast Flow from Pharmacia is washed thoroughly with Milli-Q water on a scintered glass filter. The gel is then resuspended in water to form a slurry. The slurry is poured carefully into a glass column (Pharmacia) to a volume of 6 mls (1 x 7 cm). After the gel has settled, the column is washed with 5 volumes of 50 mM EDTA (ethylenediaminetetraacetic acid) pH 8.0. Following this, the column is washed with 5 volumes of 0.2 N NaOH and 5 volumes of Milli-Q water. The column then is charged with 5 volumes of 50 mM $NiSo_4$ (or $ZnCl_2$ or $CuSO_4$). Finally, the column is washed with 5 bed volumes of equilibration buffer. The equilibration buffer is made up of 20 mM Tris pH 8.0, containing 500 mM NaCl, 1 mM PMSF, 1 mM benzamidine, 10 mg/L leupeptin, and 10 mg/L aprotinin.

The column has been equilibrated with at least 5 volumes of equilibration buffer. 5-10 mls of crude recombinant E. coli extract are applied to the column by gravity. After all the crude material has entered the column, the column is washed with 10 column volumes of equilibration buffer containing 1.0 M NaCl, instead of 500 mM NaCl, pH 8.0.

The column is then eluted with increasing concentrations of imidazole in the equilibration buffer at pH 8.0. For the earlier experiments, a large number of elutions are performed for each experiment to determine the concentration at which the chimeric is eluted. Later this elution is simplified and usually just three imidazole concentrations are used: 35 mM, 100 mM, and 300 mM imidazole in the equilibration buffer, pH 8.0. Ten bed volumes of each imidazole buffer are used. Between elutions, the column is washed with 10 volumes of equilibration buffer. Finally, the column is stripped with 5 bed volumes of 50 mM EDTA pH 8.0 to determine if any protein is still bound to the column. The flow rates for the columns are 1.0 ml/min. 5 ml fractions are collected. The columns are run at room temperature. Commercially available Pierce protein assay kits are used to determine the protein content of the samples.

Biological activity of purified Tat protein is determined by the method described incorporated herein by reference.

Conversion of the N-terminal extended fusion proteins to mature proteins

Commercially available DPP I from beef spleen (Boehringer Mannheim, Indianapolis, IN) with a specific activity of 3000 U/mg protein is used. Enzymatic conversion is carried out by incubating the chimeric protein in 20 mM tris/500 mM NaCl, pH 8, at a enzyme to substrate ratio of 1:100 (w/w) for 60 min at 25 C. The desired polypeptide is recovered from the uncleaved chimeric protein by IMAC followed by gel filtration. The authenticity is confirmed by N-terminal sequence analysis.

## Claims

### Claims for the following Contracting States : AT, BE, CH, DE, DK, FR, GB, GR, IT, LI, LU, NL, SE

1. A fusion protein represented by the formula

$$R_1\text{-(His-X)}_n\text{-}R_2$$

wherein $(His\text{-}X)_n$ represents a metal-chelating peptide;

R_1-(His-X)_n includes a dipeptidyl peptidase I substrate;
$n = 3$ to $6$;
each X is independently selected from Asp, Glu, Ala, Gly, Val, Ser, Leu, Ile and Thr;
$R_1$ is hydrogen or an even number of amino-acids free of Pro residues and not containing Arg or Lys residues at the odd-numbered positions; and
$R_2$ is a desired polypeptide or a desired polypeptide indirectly covalently linked to the metal-chelating peptide.

2. A fusion protein according to claim 1, wherein each X is independently selected from Asp and Glu.

3. A fusion protein according to claim 2, wherein each X is Asp.

4. A fusion protein according to any preceding claim, wherein $R_2$ comprises a protein selected from tPA, IL-1, bSt, IL-1 inhibitor, CD4, HIV RT, human nerve growth factor, sCD4-PE40, human respiratory syncytial virus (RSV) FG chimeric glycoprotein, and PE40.

5. A fusion protein according to any preceding claim, wherein Pro is the 2nd and/or 3rd amino-acid residue of the desired polypeptide $R_2$.

6. A method of purifying a fusion protein according to any preceding claim, comprising the steps of:

(a) contacting immobilised metal ions with cell extract material which contains cellular proteinaceous molecules including the fusion protein, wherein the fusion protein forms an affinity bond to the metal ions;
(b) removing cell extract material not bonded to the metal ions;
(c) eliminating the affinity bond; and
(d) collecting purified fusion protein.

7. A method according to claim 6, which comprises the further step of removing the metal-chelating peptide from the fusion protein by DPP I cleavage.

8. A kit for use in purifying a fusion protein according to any of claims 1 to 5, comprising:

(a) a DNA molecule which encodes the fusion protein; and
(b) a column containing immobilised metal ions.

### Claims for the following Contracting State : ES

1. A process for preparing a fusion protein represented by the formula

$$R_1\text{-(His-X)}_n\text{-}R_2$$

wherein $(His\text{-}X)_n$ represents a metal-chelating peptide;

R_1-(His-X)_n includes a dipeptidyl peptidase I substrate;
$n = 3$ to $6$;
each X is independently selected from Asp, Glu, Ala, Gly, Val, Ser, Leu, Ile and Thr;
$R_1$ is hydrogen or an even number of amino-acids free of Pro residues and not containing Arg or Lys residues at the odd-numbered positions; and
$R_2$ is a desired polypeptide or a desired polypeptide indirectly covalently linked to the metal-chelating peptide;
which comprises expression in a host transformed with a chimeric gene encoding the fusion protein, such that the host comprises (a) a promoter region, (b) a 5' untranslated region, optionally (c) a signal sequence, (d) the chimeric coding sequence, (e) a 3' untranslated region, and (f) a transcription termination site.

2. A process according to claim 1, wherein each X is independently selected from Asp and Glu.

3. A process according to claim 2, wherein each X is Asp.

4. A process according to any preceding claim, wherein $R_2$ comprises a protein selected from tPA, IL-1, bSt, IL-1 inhibitor, CD4, HIV RT, human nerve growth factor, sCD4-PE40, human respiratory syncytial virus (RSV) FG chimeric glycoprotein, and PE40.

5. A process according to any preceding claim, wherein Pro is the 2nd and/or 3rd amino-acid residue of the desired polypeptide $R_2$.

6. A method of purifying a fusion protein as defined in any preceding claim, comprising the steps of:

(a) contacting immobilised metal ions with cell extract material which contains cellular proteinaceous molecules including the fusion protein, wherein the fusion protein forms an affinity bond to the metal ions;
(b) removing cell extract material not bonded to the metal ions;
(c) eliminating the affinity bond; and
(d) collecting purified fusion protein.

7. A method according to claim 6, which comprises the further step of removing the metal-chelating peptide from the fusion protein by DPP I cleavage.

**Patentansprüche**

**Patentansprüche für folgende Vertragsstaaten : AT, BE, CH, DE, DK, FR, GB, GR, IT, LI, LU, NL, SE**

1. Fusionsprotein der Formel:

$$R_1\text{-}(His\text{-}X)_n\text{-}R_2$$

worin bedeuten:

$(His\text{-}X)_n$ ein Metallchelat bildendes Peptid;
$R_1\text{-}(His\text{-}X)_n$ ein Dipeptidylpeptidase I-Substrat;
$n = 3$ bis 6;
jedes X unabhängig voneinander Asp, Glu, Ala, Gly, Val, Ser, Leu, Ile oder Thr;
$R_1$ Wasserstoff oder eine gerade Zahl von Aminosäuren ohne Pro-Reste und ohne Arg- oder Lys-Reste an den ungeradzahligen Stellen, und

$R_2$ ein gewünschtes Polypeptid oder ein gewünschtes Polypeptid, welches indirekt kovalent an das Metallchelat bildende Peptid gebunden ist.

2. Fusionsprotein nach Anspruch 1, wobei jedes X unabhängig voneinander aus Asp und Glu ausgewählt ist.

3. Fusionsprotein nach Anspruch 2, wobei jedes X für Asp steht.

4. Fusionsprotein nach einem der vorhergehenden Ansprüche, wobei $R_2$ ein Protein, ausgewählt aus tPA, IL-1, bSt, IL-1-Inhibitor, CD4, HIV RT, Humannervenwachstumsfaktor, sCD4-PE40, humanes Atmungssynzytiumvirus (RSV) FG chimäres Glycoprotein und PE40 umfaßt.

5. Fusionsprotein nach einem der vorhergehenden Ansprüche, wobei Pro den zweiten und/oder dritten Aminosäurerest des gewünschten Polypeptids $R_2$ bildet.

6. Verfahren zum Reinigen eines Fusionsproteins nach einem der vorhergehenden Ansprüche in folgenden Stufen:

(a) Kontaktieren immobilisierter Metallionen mit zelluläre eiweißartige Moleküle einschließlich des Fusionsproteins enthaltendem Zellextraktmaterial, wobei das Fusionsprotein eine Affinitätsbindung an die Metallionen eingeht;
(b) Entfernen des nicht an die Metallionen gebundenen Zellextraktmaterials;
(c) Beseitigen der Affinitätsbindung und
(d) Sammeln von gereinigtem Fusionsprotein.

7. Verfahren nach Anspruch 6, bei welchem zusätzlich das ein Metallchelat bildende Peptid aus dem Fusionsprotein durch DPP I-Spaltung entfernt wird.

8. Besteck zur Verwendung bei der Reinigung eines Fusionsproteins nach einem der Ansprüche 1 bis 5, umfassend

(a) ein DNA-Molekül mit Codierung für das Fusionsprotein und
(b) eine immobilisierte Metallionen enthaltende Säule.

**Patentansprüche für folgenden Vertragsstaat : ES**

1. Verfahren zur Herstellung eines Fusionsproteins der Formel:

$$R_1\text{-}(His\text{-}X)_n\text{-}R_2$$

worin bedeuten:

(His-X)$_n$ ein Metallchelat bildendes Peptid;
R$_1$-(His-X)$_n$ ein Dipeptidylpeptidase I-Substrat;
n = 3 bis 6;
jedes X unabhängig voneinander Asp, Glu, Ala, Gly, Val, Ser, Leu, Ile oder Thr;
R$_1$ Wasserstoff oder eine gerade Zahl von Aminosäuren ohne Pro-Reste und ohne Arg- oder Lys-Reste an den ungeradzahligen Stellen, und
R$_2$ ein gewünschtes Polypeptid oder ein gewünschtes Polypeptid, welches indirekt kovalent an das Metallchelat bildende Peptid gebunden ist,
durch Expression in einem mit einem chimären Gen mit Codierung für das Fusionsprotein derart transformierten Wirt, daß letzterer
(a) einen Promotorbereich,
(b) einen 5'-untranslatierten Bereich, gegebenenfalls
(c) eine Signalsequenz,
(d) die chimäre Codiersequenz,
(e) einen 3'-untranslatierten Bereich und
(f) eine Transkriptionsterminationsstelle umfaßt.

2. Verfahren nach Anspruch 1, wobei jedes X unabhängig voneinander aus Asp und Glu ausgewählt ist.

3. Verfahren nach Anspruch 2, wobei jedes X aus Asp besteht.

4. Verfahren nach einem der vorhergehenden Ansprüche, wobei R$_2$ ein Protein, ausgewählt aus tPA, IL-1, bSt, IL-1-Inhibitor, CD4, HIV RT, Humannervenwachstumsfaktor, sCD4-PE40, humanes Atmungssynzytiumvirus (RSV) FG chimäres Glycoprotein und PE40, umfaßt.

5. Verfahren nach einem der vorhergehenden Ansprüche, wobei Pro den zweiten und/oder dritten Aminosäurerest des gewünschten Polypeptids R$_2$ bildet.

6. Verfahren zum Reinigen eines Fusionsproteins nach einem der vorhergehenden Ansprüche in folgenden Stufen:

(a) Kontaktieren immobilisierter Metallionen mit zelluläre eiweißartige Moleküle einschließlich des Fusionsproteins enthaltendem Zellextraktmaterial, wobei das Fusionsprotein eine Affinitätsbindung an die Metallionen eingeht;
(b) Entfernen des nicht an die Metallionen gebundenen Zellextraktmaterials;
(c) Beseitigen der Affinitätsbindung und

(d) Sammeln von gereinigtem Fusionsprotein.

7. Verfahren nach Anspruch 6, bei welchem zusätzlich das ein Metallchelat bildende Peptid aus dem Fusionsprotein durch DPP I-Spaltung entfernt wird.

## Revendications

**Revendications pour les Etats contractants suivants : AT, BE, CH, DE, DK, FR, GB, GR, IT, LI, LU, NL, SE**

1. Protéine de fusion représentée par la formule

$$R_1\text{-(His-X)}_n R_2$$

dans laquelle (His-X)$_n$ représente un peptide chélatant les métaux ;

R$_1$-(His-X)$_n$ comprend un substrat de dipeptidyl-peptidase I ;
n a une valeur de 3 à 6 ;
chaque groupe X est choisi, indépendamment, entre Asp, Glu, Ala, Gly, Val, Ser, Leu, Ile et Thr ;
R$_1$ représente l'hydrogène ou un nombre pair d'amino-acides dépourvus de résidus Pro et ne contenant pas de résidus Arg ou Lys aux positions impaires ; et
R$_2$ représente un polypeptide désiré ou un polypeptide désiré lié indirectement par covalence au peptide chélatant les métaux.

2. Protéine de fusion suivant la revendication 1, dans laquelle chaque groupe X est choisi, indépendamment, entre Asp et Glu.

3. Protéine de fusion suivant la revendication 2, dans laquelle chaque groupe X représente Asp.

4. Protéine de fusion suivant l'une quelconque des revendications précédentes, dans laquelle R$_2$ comprend une protéine choisie entre le tPA, la Il-1, la bSt, l'inhibiteur de IL-1, la protéine CD4, la protéine RT de HIV, le facteur de croissance neurale humain, la protéine sCD4-PE40, la glycoprotéine chimère FG de virus syncytial respiratoire (RSV) humain et la protéine PE40.

5. Protéine de fusion suivant l'une quelconque des revendications précédentes, dans laquelle Pro représente le 2ème et/ou le 3ème résidu d'amino-acide du polypeptide R$_2$ désiré.

6. Procédé de purification d'une protéine de fusion suivant l'une quelconque des revendications précédentes, comprenant les étapes consistant :

(a) à mettre en contact des ions métalliques immobilisés avec un extrait cellulaire qui contient des molécules protéiques cellulaires comprenant la protéine de fusion, dans lequel la protéine de fusion forme une liaison d'affinité avec les ions métalliques ;

(b) à éliminer l'extrait cellulaire non lié aux ions métalliques ;

(c) à supprimer la liaison d'affinité ; et

(d) à recueillir la protéine de fusion purifiée.

7. Procédé suivant la revendication 6, qui comprend l'étape supplémentaire consistant à séparer le peptide chélatant les métaux de la protéine de fusion par clivage avec la DPP I.

8. Kit destiné à être utilisé dans la purification d'une protéine de fusion suivant l'une quelconque des revendications 1 à 5, comprenant :

(a) une molécule d'ADN qui code pour la protéine de fusion ; et

(b) une colonne contenant des ions métalliques immobilisés.

**Revendications pour l'Etat contractant suivant : ES**

1. Procédé de préparation d'une protéine de fusion représentée par la formule

$$R_1\text{-}(His\text{-}X)_n R_2$$

dans laquelle $(His\text{-}X)_n$ représente un peptide chélatant les métaux ;

$R_1\text{-}(His\text{-}X)_n$ comprend un substrat de dipeptidyl-peptidase I

$\underline{n}$ a une valeur de 3 à 6 ;

chaque groupe X est choisi, indépendamment, entre Asp, Glu, Ala, Gly, Val, Ser, Leu, Ile et Thr ;

$R_1$ représente l'hydrogène ou un nombre pair d'amino-acides dépourvus de résidus Pro et ne contenant pas de résidus Arg ou Lys aux positions impaires ; et

$R_2$ représente un polypeptide désiré ou un polypeptide désiré lié indirectement par covalence au peptide chélatant les métaux ; qui comprend l'expression dans un hôte transformé avec un gène chimère codant pour la protéine de fusion, de telle sorte que l'hôte comprenne (a) une région de promoteur, (b) une région non traduite en 5', facultativement (c) une séquence signal, (d) la séquence codante chimère, (e) une région non traduite en 3', et (f) un site de terminaison de transcription.

2. Procédé suivant la revendication 1, dans lequel chaque groupe X est choisi, indépendamment, entre Asp et Glu.

3. Procédé suivant la revendication 2, dans lequel chaque groupe X représente Asp.

4. Procédé suivant l'une quelconque des revendications précédentes, dans lequel $R_2$ comprend une protéine choisie entre le tPA, la Il-1, la bSt, l'inhibiteur de IL-1, la protéine CD4, la protéine RT de HIV, le facteur de croissance neurale humain, la protéine sCD4-PE40, la glycoprotéine chimère FG de virus syncytial respiratoire (RSV) humain et la protéine PE40.

5. Procédé suivant l'une quelconque des revendications précédentes, dans lequel le résidu Pro est le 2ème et/ou le 3ème résidu d'amino-acide du polypeptide $R_2$ désiré.

6. Procédé de purification d'une protéine de fusion répondant à la définition suivant l'une quelconque des revendications précédentes, comprenant les étapes consistant :

(a) à mettre en contact des ions métalliques immobilisés avec un extrait cellulaire qui contient des molécules protéiques cellulaires comprenant la protéine de fusion, dans lequel la protéine de fusion forme une liaison d'affinité avec les ions métalliques ;

(b) à éliminer l'extrait cellulaire non lié aux ions métalliques ;

(c) à supprimer la liaison d'affinité ; et

(d) à recueillir la protéine de fusion purifiée.

7. Procédé suivant la revendication 6, qui comprend l'étape supplémentaire de séparation du peptide chélatant les métaux de la protéine de fusion par clivage avec la DPP I.